# EUROPEAN PATENT APPLICATION

(11) **EP 4 512 301 A1**
(43) Date of publication of application: **26.02.2025**
(21) Application number: 23923042.8
(22) Date of filing: 26.10.2023
(51) Int. Cl.: A47L 23/20, A61L 2/07, A61L 2/24, A47B 61/04, F26B 3/02, F26B 21/00, F26B 21/06, F26B 21/08

(54) **SHOE CARE APPARATUS**

(30) Priority: 14.02.2023 KR 20230019529
(71) Applicant: LG Electronics Inc., Yeongdeungpo-gu Seoul 07336 (KR)
(72) Inventor: KIM, Jong Hwan, Seoul 08592 (KR)
(74) Representative: Schornack, Oliver
(86) International application number: PCT/KR2023/016693
(87) International publication number: WO 2024/172243

(57) **Abstract**

Provided is a shoe care device in which a shoe is processed by circulating air flow. The shoe care device according to one aspect of the present invention is capable of executing a processing stroke on a shoe accommodated in an accommodation space of an inner cabinet. The shoe care device includes: a connection path configured to provide a flow path into which air from the accommodation space is introduced and then discharged back into the accommodation space; a blowing part disposed in the connection path and configured to blow air; a dehumidifying part disposed in the connection path and configured to dehumidify the air; a steam part configured to supply steam to the inner cabinet; a control panel disposed outside the shoe care device so that a control signal for the processing process can be input by a user; and a controller configured to control a display state of information output through the control panel depending on a type of the processing stroke. The control panel includes: a sub-layer where a first control UI, of which display-on/off can be controlled, is disposed; and a main layer where a second control UI, of which display-on/off and type can be controlled, is disposed.

## Description

### TECHNICAL FIELD

The present invention relates to a shoes care device, and more particular, to a shoes care device which treats shoes by air circulation.

### BACKGROUND

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

According to Prior Document 1 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 1 above, the air from which moisture has been removed by a deodorization module including zeolite and activated carbon can be discharged to the outside of the apparatus for sterilization disposal of shoes.

However, in Prior Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

According to Prior Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 2 above, the air is circulated by a circulating fan in a shoe closet, and the sanitary filter portion is disposed on a circulation path of the air to remove germs and odors in the air.

However, since Prior Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience. There is a need to develop a shoes care device that takes these matters into consideration.

In addition, the reduction of manufacturing costs, the convenience of manufacturing and assembling each component, and the convenience of maintenance needs to be considered in the development of the shoes care device.

### DISCLOSURE

### TECHNICAL PROBLEM

An object to be achieved by the present invention is to solve the aforementioned problems caused by a shoes care device which treats shoes by air circulation.

Specifically, an object to be achieved by the present invention is to provide a shoes care device which can ensure proper performance for shoes treatment at all times by performing dehumidification and deodorization on the shoes using a dehumidifying material to refresh the shoes and regenerating the used dehumidifying material.

An object to be achieved by the present invention is to provide a shoes care device which prevents air used for dehumidification and deodorization of shoes from being exposed to a user by an air circulation structure capable of dehumidifying the air inside an inner cabinet where the shoes are placed by using a dehumidifying material and supplying the dehumidified air again to the inner cabinet.

An object to be achieved by the present invention is to provide a shoe care device in which, when a control signal is input by a user, the display of output information can be optimally controlled depending on the type of a processing stroke.

The technical objects of the present disclosure are not restricted to those set forth Here, and other unmentioned technical objects will be clearly understood by one of ordinary skill in the art to which the present disclosure pertains by referencing the detailed description of the present disclosure given below.

### TECHNICAL SOLUTION

In order to achieve the above and other objects, a shoes care device according to one aspect of the present invention is configured not only to perform dehumidification and deodorization of shoes using a dehumidifying part but also to regenerate the used dehumidifying part. Specifically, the shoes care device is configured not only to collect moisture and bacteria in air ventilated by the dehumidifying part in a module chamber but also to heat and regenerate the dehumidifying part in the module chamber.

In addition, according to one aspect of the present invention, the shoes care device is configured such that the air used for dehumidifying and deodorizing the shoes has an air circulation structure inside the shoes care device. Specifically, a connection path through which air is circulated is formed between a outlet and a nozzle disposed inside an inner cabinet, respectively.

In addition, according to one aspect of the present invention, the shoe care device is configured such that the display state of information output to a user is controlled depending on a control signal for a processing stroke input by the user. Specifically, when a user inputs a control signal for a processing stroke to a control panel, the display state of information output through the control panel is controlled accordingly.

In addition, according to one aspect of the present invention, the shoe care device may include a plurality of inner cabinets, and UIs for respective inner cabinets may be individually displayed on a main layer.

In addition, according to one aspect of the present invention, in the shoe care device, the plurality of inner cabinets may be arranged in a vertical direction, and UIs for respective inner cabinets may be divided and displayed up and down in the main layer.

In addition, in the shoe care device according to one aspect of the present invention, by selecting a setting UI, it is possible to check on the main layer whether the processing strokes of respective inner cabinets will be executed independently of each other.

In addition, in the shoe care device according to one aspect of the present invention, at least one of a stroke type, a remaining time, a progress rate, and detailed stroke information for each inner cabinet may be displayed on the main layer.

In addition, in the shoe care device according to one aspect of the present invention, the state of the inner cabinet for which the processing stroke was completed first may be displayed on the main layer until all the processing strokes are completed.

In addition, in the shoe care device according to one aspect of the present invention, when the processing strokes of respective inner cabinets are equal to each other, UIs for respective inner cabinets may be integrated and displayed on the main layer.

In addition, in the shoe care device according to one aspect of the present invention, when there is no additional operation for a set period of time while the processing stroke is being executed, the remaining time may be displayed in an enlarged format on the main layer.

In addition, in the shoe care device according to one aspect of the present invention, the type of a UI for each inner cabinet may be checked on the main layer in a sliding manner.

In addition, in the shoe care device according to one aspect of the present invention, a viewing UI may be selected to check a plurality of types of UIs for each inner cabinet on the main layer.

In addition, in the shoe care device according to one aspect of the present invention, while a processing stroke is being executed, an operation UI may be selected to check whether to immediately terminate the processing stroke on the main layer.

In addition, in the shoe care device according to one aspect of the present invention, a setting UI may be selected to check the completion time point of a processing stroke to be reserved on the main layer.

In addition, in the shoe care device according to one aspect of the present invention, the setting UI may be selected to check on the main layer whether to maintain the setting UI as a storage state after the processing stroke is completed.

In addition, in the shoe care device according to one aspect of the present disclosure, the types of UIs may be sequentially arranged depending on the frequencies of processing strokes executed a set number of times or more.

In addition, in the shoe care device according to one aspect of the present invention, the setting UI may be selected to check a UI selection list on the main layer.

The technical objects of the present disclosure are not restricted to those set forth Here, and other unmentioned technical objects will be clearly understood by one of ordinary skill in the art to which the present disclosure pertains by referencing the detailed description of the present disclosure given below.

### BRIEF DESCRIPTION OF THE DRAWINGS

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together.
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12 is a view illustrating in more detail the control panel in the shoe care device according to one embodiment of the present invention.
FIG. 13 is a view illustrating a UI implemented in the shoe care device according to one embodiment of the present invention.
FIG. 14 is a view illustrating, by way of an example, the state in which general care and dual care are selected through a setting UI in the shoe care device according to one embodiment of the present invention.
FIGS. 15 and 16 are views illustrating, by way of an example, UIs which are operated in a dual care mode in the shoe care device according to one embodiment of the present invention.
FIGS. 17 and 18 are views illustrating, by way of an example, the state in which an operation UI is selected while a processing stroke is being executed in the shoe care device according to one embodiment of the present invention.
FIG. 19 is a view illustrating, by way of an example, the state of the main layer displayed when the setting UI is selected in the shoe care device according to one embodiment of the present invention.
FIG. 20 is a view illustrating, by way of an example, the state in which a completion time point of a processing stroke is reserved through the setting UI in the shoe care device according to one embodiment of the present invention.
FIG. 21 is a view illustrating, by way of an example, the state in which shoes are stored after completion of a processing stroke through the setting UI in the shoe care device according to one embodiment of the present invention.
FIG. 22 is a view illustrating, by way of an example, the state in which the types of UIs are arranged depending on frequencies of processing strokes in the shoe care device according to one embodiment of the present invention.
FIG. 23 is a view illustrating, by way of an example, the state in which a UI selection list is displayed on the main layer in the shoe care device according to one embodiment of the present invention.

### DETAILED DESCRIPTION

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

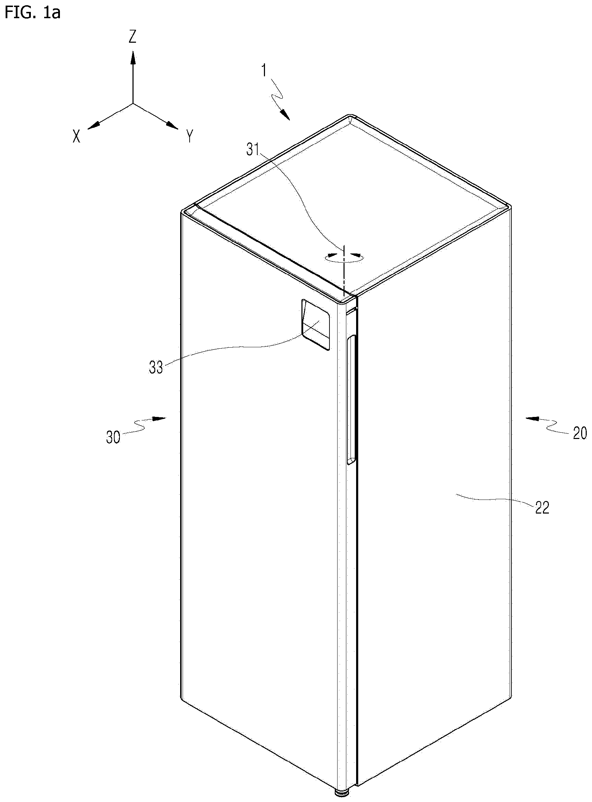

FIG. 1a is a perspective view illustrating a shoes care device according to one embodiment of the present invention.

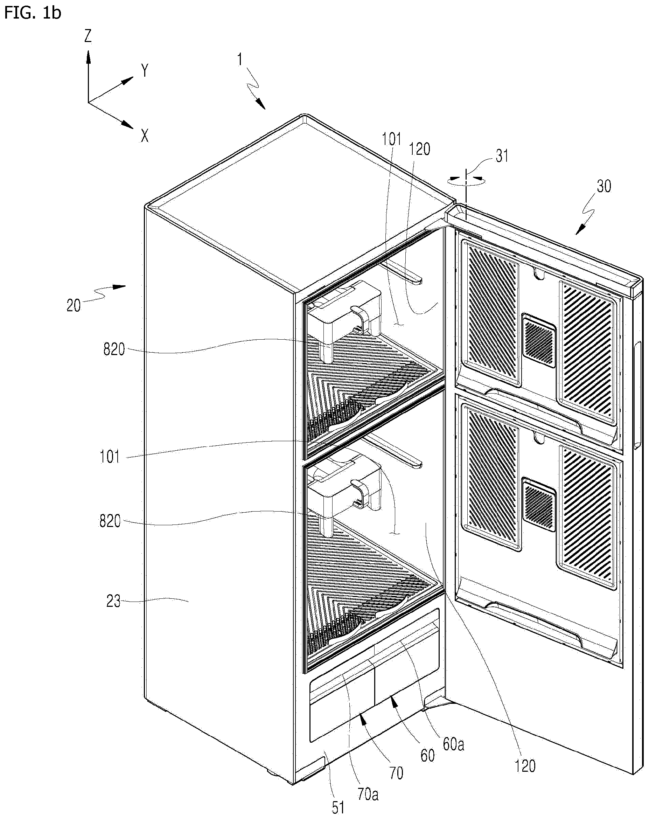

FIG. 1b is a perspective view of the shoes care device of FIG. 1a, when viewed from another direction, illustrating a state in which a door is opened.

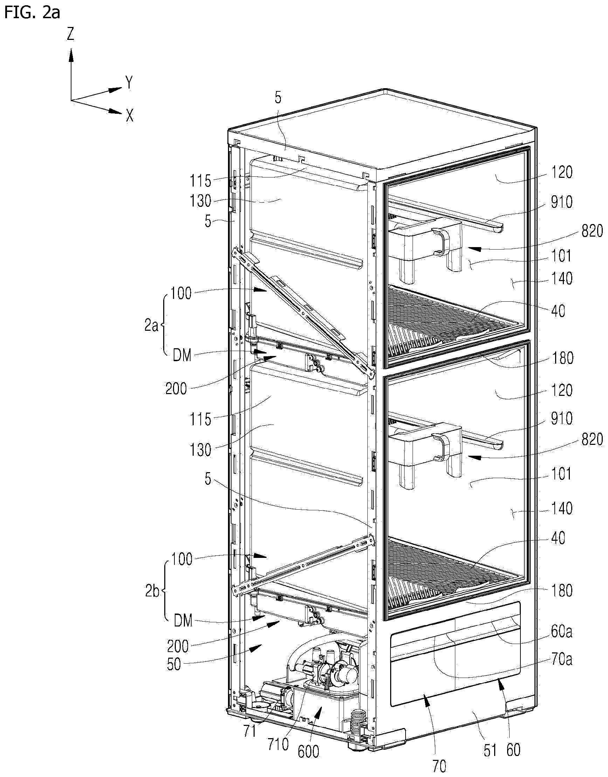

FIG. 2a is a perspective view illustrating a state in which a part of the door and an outer cabinet are removed from the shoes care device of FIG. 1b.

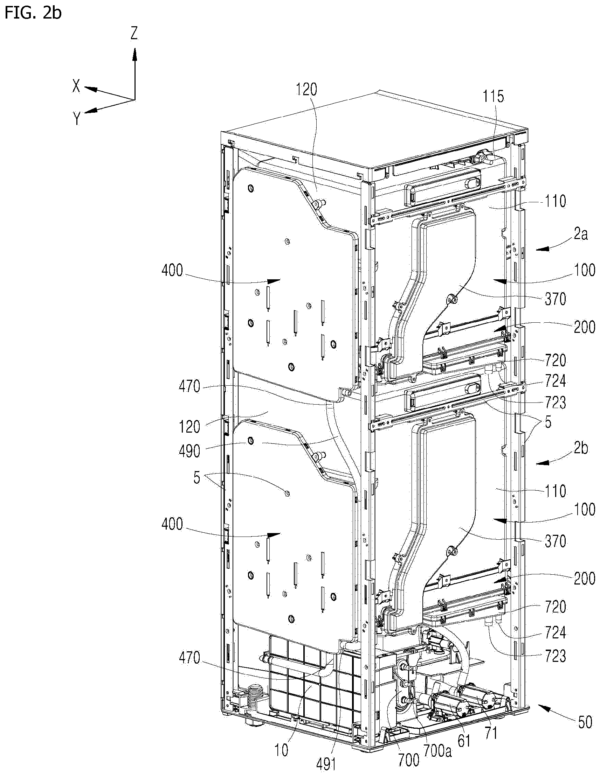

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIGS. 53 and 54).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

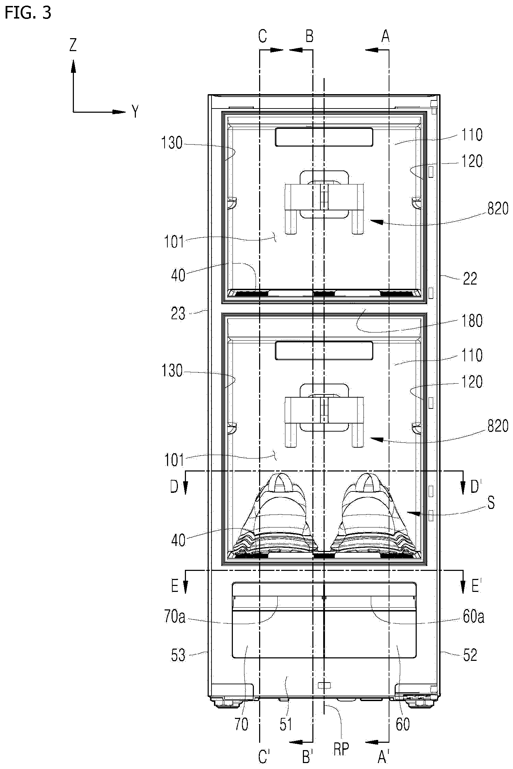

FIG. 3 is a front view illustrating a state in which a door is removed from the shoes care device illustrated in FIG. 1b. FIG. 3 illustrates shoes accommodated in an inner cabinet together.

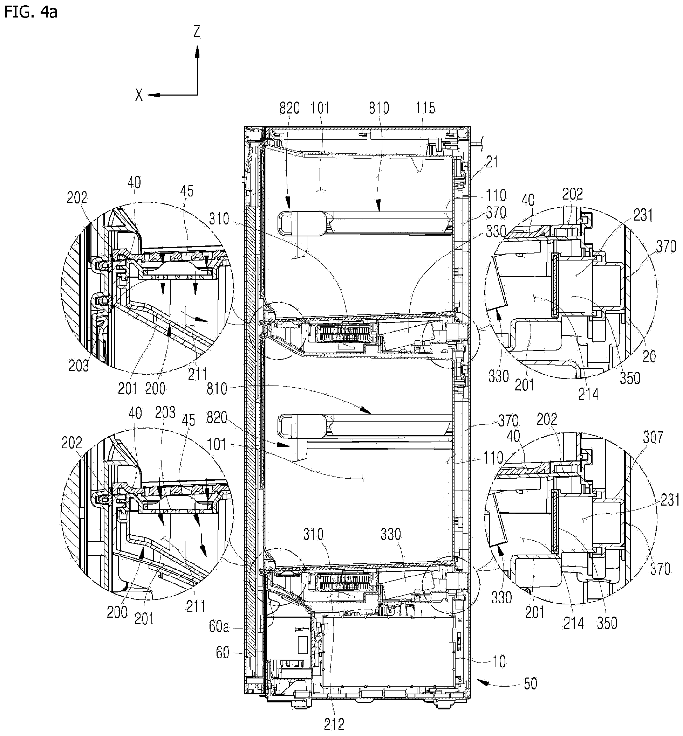

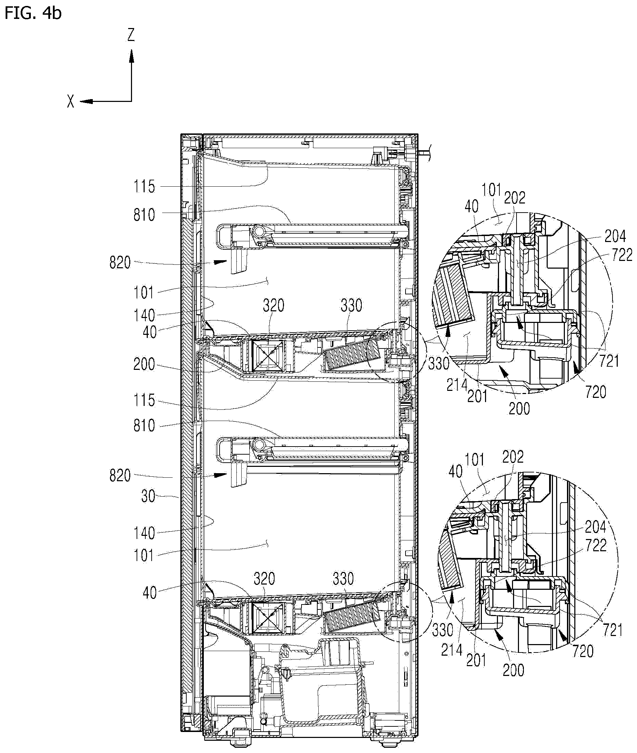

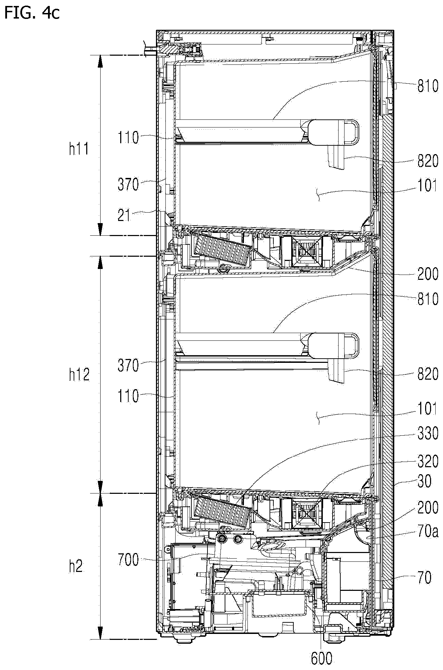

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include a outlet 203 and a nozzle 820.

The management devices 2a and 2b may include the module housing 200 forming a connection path F10.

The management devices 2a and 2b may include a regeneration path F20. The management devices 2a and 2b may include a heating part 320.

The management devices 2a and 2b may include a conversion flow path F10a.

The management devices 2a and 2b may include a damper 350.

The shoes care device 1 may include a steam part 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoes care device 1 moves.

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam part 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam part 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

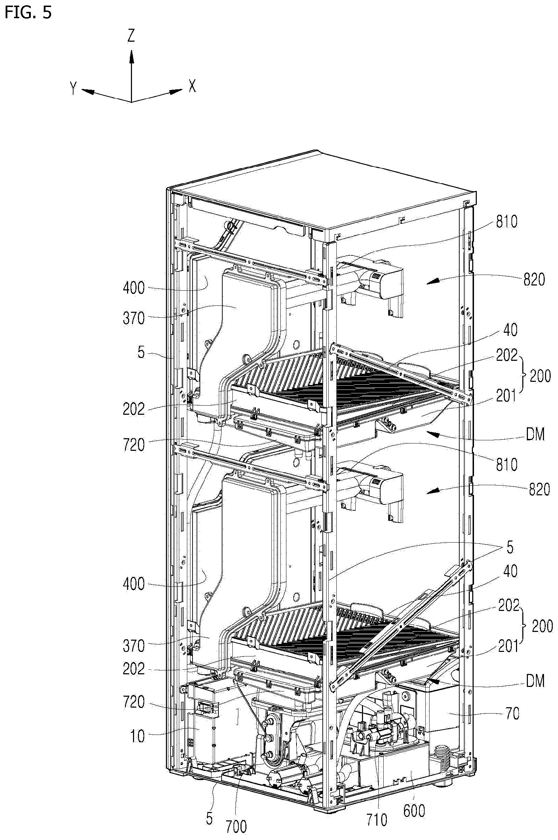

FIG. 5 is a perspective view illustrating a state in which the door 30, the outer cabinet 20, and the inner cabinet 100 are removed from the shoes care device 1 according to one embodiment of the present invention.

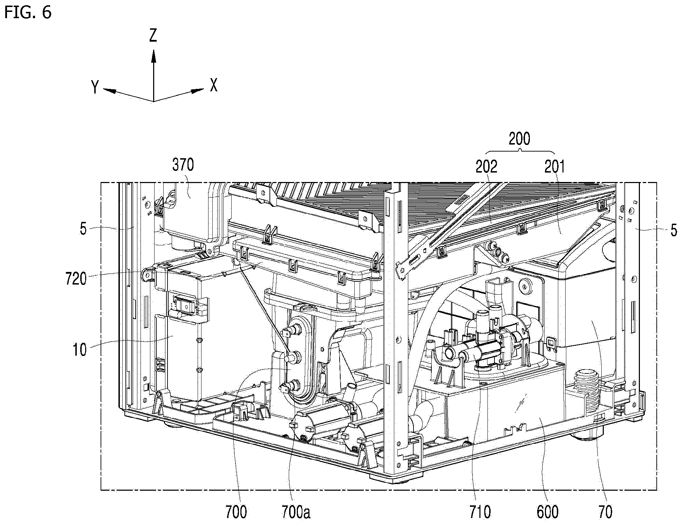

FIG. 6 is a perspective view illustrating a machine room part of the shoes care device 1 illustrated in FIG. 5.

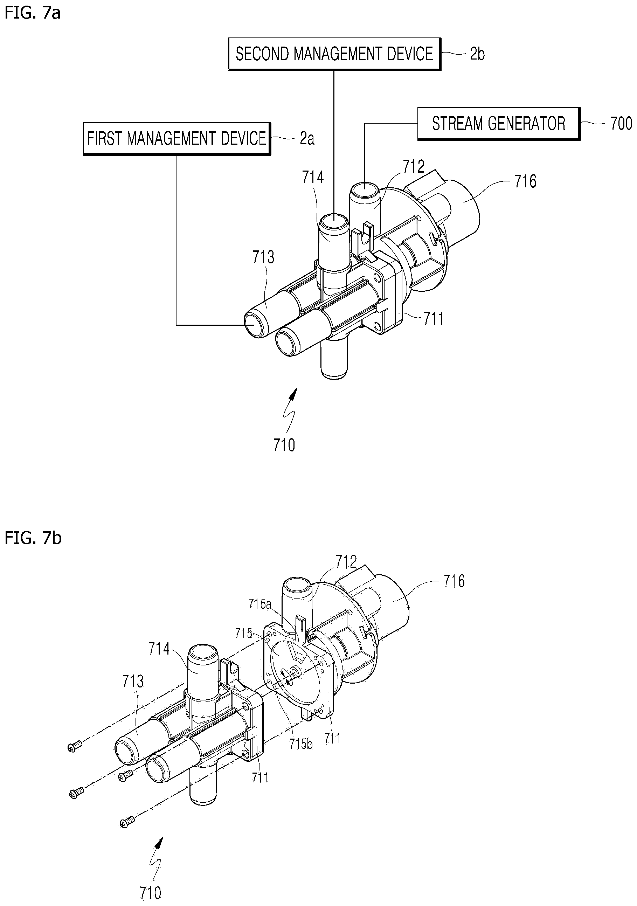

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam part 700 as a device configured so as to generate moisture inside the inner cabinet 100. The steam part 700 may be provided inside the machine room 50. The steam part 700 is configured to generate steam and selectively supply moisture and steam to the inside of the inner cabinet 100.

The shoes care device 1 according to one embodiment may be provided with one steam part 700, and the shoes care device 1 according to another embodiment may be provided with two or more steam parts 700.

When the shoes care device 1 is provided with one steam part 700, the steam part 700 may be configured to supply steam into the inner cabinet 100 of the first management device 2a and/or into the inner cabinet 100 of the second management device 2b.

When the shoes care device 1, one steam part 700 may be provided with two or more steam parts 700, one of the steam parts 700 may supply steam to the inner cabinet 100 of the first management device 2a, and the other steam part 700 may supply steam to the inner cabinet 100 of the second management device 2b.

Moist air formed by the steam part 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam part 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam part 700 may include a device such as a steam generator, in which the steam part may be provided with an inner space and a separate heater 700a configured to heat water in the inner space, and configured to heat the water to generate steam and supply the steam to the accommodation space 101 of the inner cabinet 100. In addition, even if separate devices such as a steam generator are not included, when heating the dehumidifying part 330, the steam part 700 may use relatively high temperature moisture separated from the dehumidifying part 330 as steam and supply the moisture into the accommodation space 101 of the inner cabinet 100.

An external faucet, etc., as a water supply source for supplying water to the steam part 700, may be used, or a container-type water supply tank provided on one side of the machine room 50 may be used. The steam part 700 may generate steam by receiving water from the water supply tank 60.

The water supply tank 60 for the movement of water and the steam part 700 may be connected by a pipe, a hose, etc.

The steam part 700 for the movement of steam and the inner cabinet 100 may be connected by a pipe, a hose, etc. In the shoes care device 1 according to one embodiment of the present invention, as described below, the steam generated by the steam part 700 may be supplied into the inner cabinet 100 after passing through the steam valve 710 and a steam separator 720. In this case, in order to move the steam, the steam part 700 and the steam valve 710 may be connected by a pipe, a hose, etc., and the steam valve 710 and the steam separator 720 may also be connected by a pipe, a hose, etc.

The steam valve 710 may be disposed adjacent to the steam part 700, and the steam valve 710 may be provided in the machine room 50. The steam part 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam part 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam part 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam part 700 may be supplied to the accommodation space 101 of the first management device 2a, or may be supplied to the accommodation space 101 of the second management device 2b. In this case, the steam generated by the steam part 700 may be supplied to the accommodation space 101 of the first management device 2a or the accommodation space 101 of the second management device 2b without a pressure drop, and even when only one steam part 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam part 700 may be selectively or simultaneously supplied to the accommodation space 101 of the first management device 2a and the accommodation space 101 of the second management device 2b, and whether the steam is supplied or not may be controlled according to a use state of the shoes care device 1.

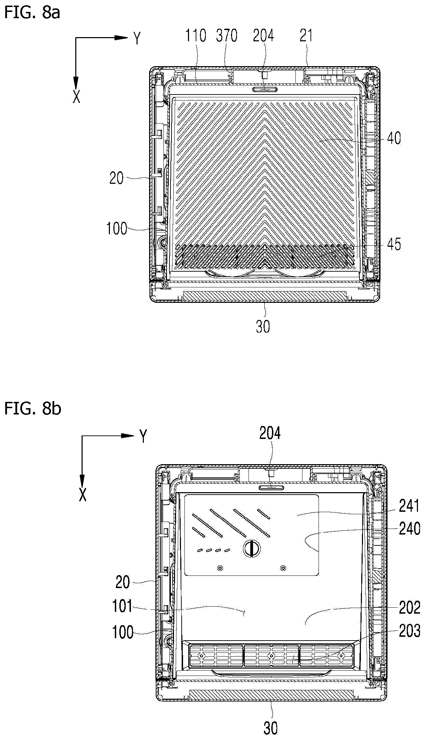

FIG. 8a is a cross-sectional view taken along line D-D' of the shoes care device 1 illustrated in FIG. 3.

FIG. 8b is a view illustrating a state in which the main shelf 40 is removed from the shoes care device 1 illustrated in FIG. 8a.

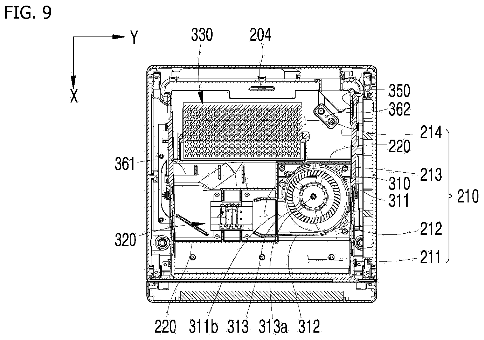

FIG. 9 is a cross-sectional view taken along line E-E' of the shoes care device 1illustrated in FIG. 3.

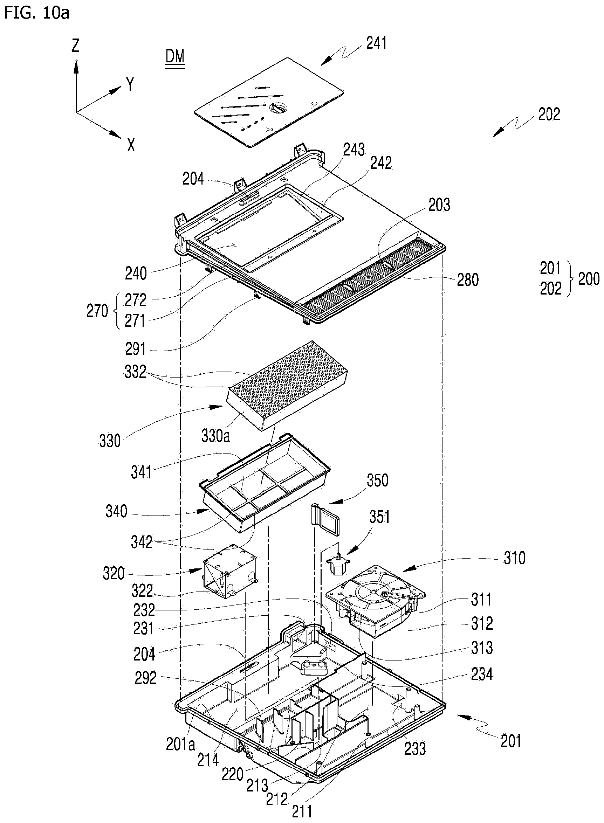

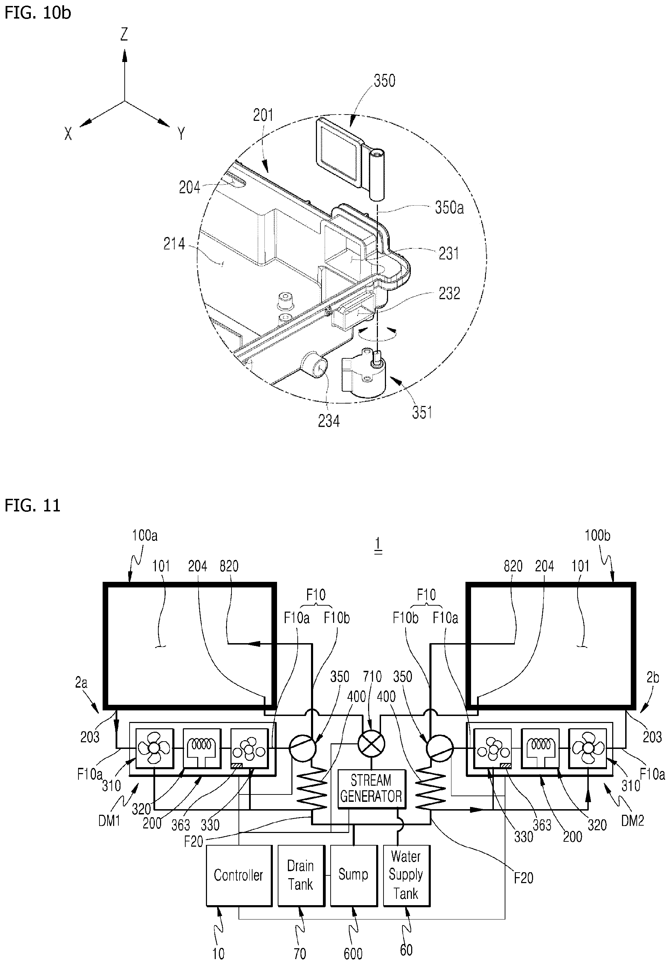

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air.

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (Å) are regularly arranged, and may function as dehumidification by adsorbing the moisture in the air.

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of the blowing part 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed.

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user.

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may reproduce bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoes care device 1 according to one embodiment of the present invention, steam generated by the steam part 700 is supplied to the accommodation space 101 of the inner cabinet 100, and to this end, the shoes care device 1 includes a steam inlet 204.

The steam inlet 204 forms an inlet through which steam is supplied to the accommodation space 101 of the inner cabinet 100.

In the shoes care device according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam part 700 and the steam valve 710 are disposed in a lower part, the distance from the steam part 700 and the steam valve 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam part 700 to the steam inlet 204

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the blowing part 310, the heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 (the dehumidifying material 331) are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration path F20 forms a passage through which air and/or condensed water inside the shoes care device moves.

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam part 700.

In the shoes care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path F10a and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam part 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam part 700, the steam part 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

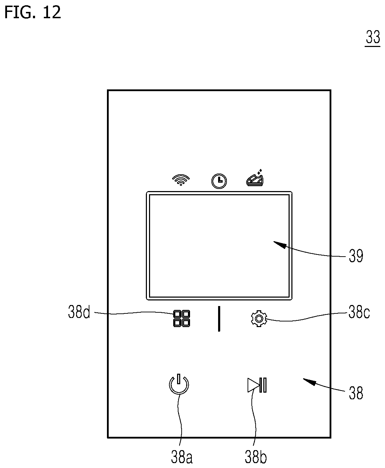

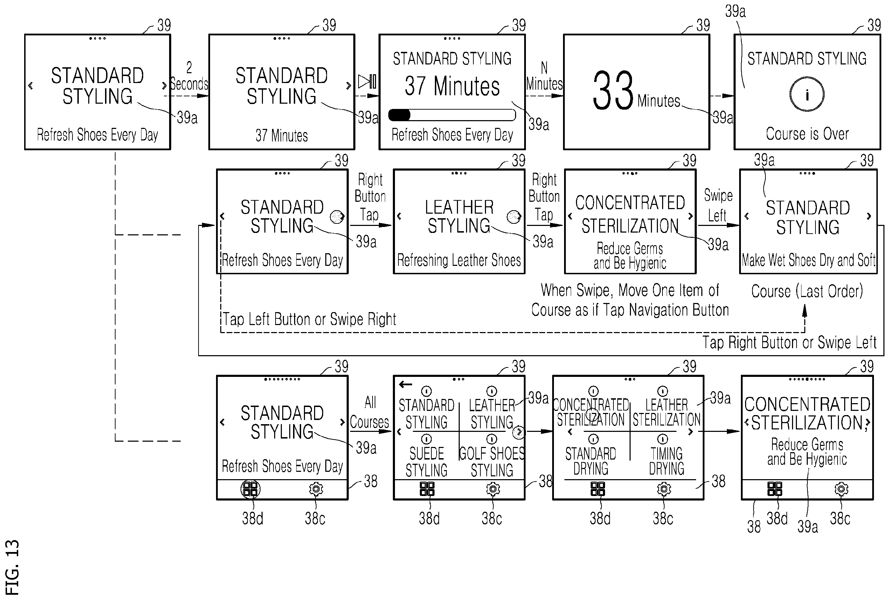

FIG. 12 is a view illustrating in more detail the control panel 33 in the shoe care device 1 according to one embodiment of the present invention. FIG. 13 is a view illustrating a UI implemented in the shoe care device 1 according to one embodiment of the present invention.

The shoe care device 1 according to one embodiment of the present invention may include an inner cabinet 100, a connection path F10, a blowing part 310, a dehumidifying part 330, a steam part 700, a control panel 33, and a controller 10.

The inner cabinet 100 is a part that is provided with the accommodation space 101 configured to accommodate shoes. An outlet 203 and a nozzle 820 may be disposed in the inner cabinet 100 to execute a processing stroke on shoes accommodated in the accommodation space 101.

The connection path F10 is a part that forms a flow path through which air from the accommodation space 101 is introduced and then discharged back to the accommodation space 101. The connection path may be an air flow path through which the air inside the inner cabinet 100 is dehumidified while being suctioned into the module chamber 210 and blown to pass through the dehumidifying part 330 and is supplied back into the inner cabinet 100.

The blowing part 310 is a part disposed in the connection path F10 and configured to blow air. When the blowing part 310 is operated, air may be suctioned from the inner cabinet 100, and the suctioned air may be blown from the connection path F10.

The dehumidifying part 330 is a part disposed in the connection path F10 and configured to dehumidify air. With the heating of the dehumidifying material 430, the moisture adsorbed on the dehumidifying material 430 may be separated and the dehumidifying material 430 may be regenerated to the state in which the dehumidifying function can be performed.

The module chamber 210 is a part configured to blow air of the accommodation space 210, disposed on the air path through the dehumidifying part 330, and configured to be capable of heating the dehumidifying part 330.

In this case, when the dehumidifying part 330 is heated, the moisture adsorbed on the dehumidifying part 330 may be separated, and the dehumidifying part 330 may be regenerated to the state in which the dehumidifying function can be performed.

To this end, the module chamber 210 may include the dehumidifying part 330, the blowing part 310, the heating part 320, and a damper 350.

In particular, the module chamber 210 may form a portion of the connection path F10, through which air circulates between the outlet 203 and the nozzle 820, and the regeneration path F20, which is branched from the connection path F10 passing through the dehumidifying part 330, so that blowing is performed therethrough.

Accordingly, the air in the accommodation space 101 may move to the connection path F10 or the regeneration path F20 in the process of being blown to the module chamber 210 to pass through the dehumidifying part 330.

In this way, the shoe care device 1 according to the present embodiment may always maintain appropriate shoe processing performance since the dehumidifying part 330 is disposed in the module chamber 210 to collect moisture and bacteria in the blown air and to be regenerated by being heated in the module chamber 210.

In addition, the shoe care device 1 according to the present embodiment may prevent a user from being exposed to the air used to dehumidify and deodorize shoes since the connection path F10 through which air circulates is provided between the outlet 203 and the nozzle 820 each of which is disposed inside the inner cabinet 100.

The steam part 700 is a part configured to supply steam to the inner cabinet 100. Since a steam treatment stroke on shoes can be executed by supplying steam into the inner cabinet 100 through the steam part 700, a refreshing effect due to swelling of the shoe material or the like as well as a sterilization effect due to the high temperature of the steam can be achieved.

The control panel 33 is a part disposed outside the shoe care device 1 to allow a user to input a control signal for a processing stroke. The user may input an operation signal for each of the components of the shoe care device 1 by operating the control panel 33.

In this case, the control panel 33 may include a touch screen. A control unit (the controller 10) configured to control each of the components of the shoe care device 1 in association with the control panel 33 may be provided in the inner space of the shoe care device 1. The controller 10 may be provided inside a machine room 50.

In addition, a user interface (UI) may be placed on the control panel 33, and a user may input a control signal for a processing stroke of the shoe care device 1 by operating the UI placed on the control panel 33.

In this case, the control panel 33 may include a sub-layer 38 and a main layer 39.

The sub-layer 38 is a part where first control UIs 38a, 38b, 38c, and 38d, of which the display-on/off is controllable, are arranged, and the main layer 39 is a part where a second control UI, of which the display-on/off and type are controllable, is arranged.

In this case, the first control UIs 38a, 38b, 38c, and 38d are UIs which are essentially used due to the characteristics thereof but do not require a change in their types, like a power UI 38a, an operation UI 38b, a setting UI 38c, and a viewing UI 38d. The user may recognize these UIs as always being placed at those positions on the control panel 33.

Here, the power UI 38a may be used to operate the power on/off of the shoe care device 1, the operation UI 38b may be used to operate start/stop of the operation of the shoe care device 1, the setting UI 38c may be used to select the setting of the shoe care device 1, and the viewing UI 38d may be used to select the stroke display of the shoe care device 1.

The second control UI 39a needs to be used while being changed to various types, such as a stroke UI, a material UI, and a time UI, due to its characteristics, and the user needs to operate the second control UI while checking the state and type of the UI that is currently being implemented.

Here, the stroke UI may be used to set the type of the stroke to be executed on shoes in the shoe care device 1, the material UI may be used to select the material of the shoes to be processed in the shoe care device 1 and to enable optimal processing to be executed accordingly, and the time UI may be used to set a processing time for processing shoes in the shoe care device 1.

Meanwhile, when the user selects a first control UI 38a, 38b, 38c, or 38d, the second control UI 39a linked thereto may be displayed and changed on the main layer 39.

The controller 10 is a part that controls the display state of information output through the control panel 33 depending on the type of a processing stroke, and may change the display state of the UI depending on a control signal input by the user.

That is, depending on the type of the processing stroke intended by the user, the control panel 33 may be controlled by the controller 10 to output the display, progress state, related information, and the like of a corresponding stroke in a matching manner.

In this way, in the shoe care device 1 according to the present embodiment, when the user inputs a control signal for a processing stroke to the control panel 33, the display state of the information output through the control panel 33 is controlled accordingly. Therefore, the user may easily check the state of the shoe manager 1 and input a control signal for the intended processing stroke smoothly.

Hereinafter, the state in which UIs are displayed and changed in relation to the operation of the shoe care device 1 will be described with reference to FIG. 13.

First, a second control UI 39a for a specific processing stroke may be displayed on the control panel 33 (especially, the main layer 39) such that the user can select an intended processing stroke.

In this case, when the user swipes the screen of the main layer 39 or taps the left or right button displayed on the screen of the main layer 39, the UI may be changed into a second control UI 39a for another specific processing stroke.

Alternatively, when the user selects the viewing UI 38d, a plurality of second control UIs 39a for respective processing strokes may be displayed on the main layer 39 at once, and when the user select one of the second control UIs, the display may be changed to a second control UI 39a for another specific processing stroke.

In this case, the number of second control UIs 39a displayed per page of the main layer 39 may be set arbitrarily, and when the user swipes the screen of the main layer 39 or taps the left or right button displayed on the screen of the main layer 39, a plurality of second control UIs 39a on different pages of the main layer 39 may be displayed at once.

Through this, the user can select the intended processing stroke, and the corresponding processing stroke may be initiated by selecting the operation UI 38b in the state in which the second control user 39a for the processing stroke is selected.

In addition, when the corresponding processing stroke is completed, it may be displayed on the main layer 39 that the corresponding processing stroke has been completed.

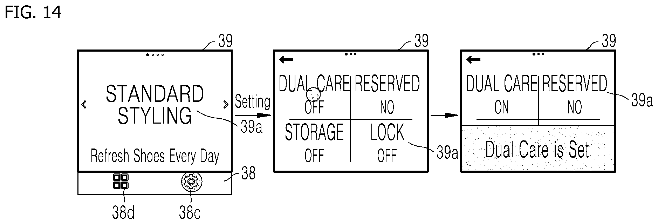

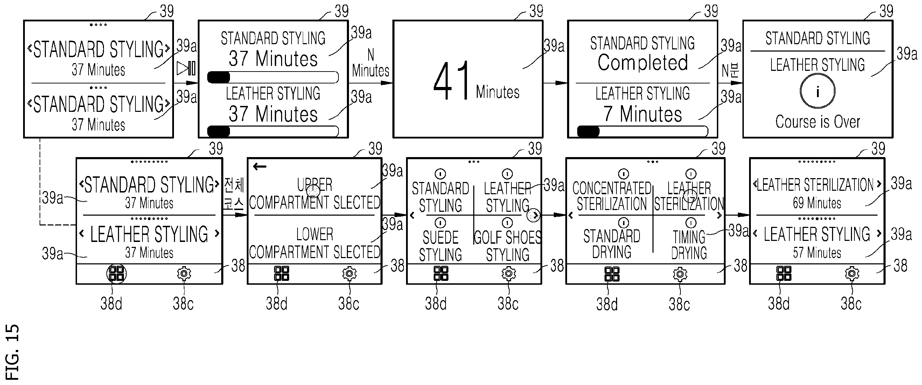

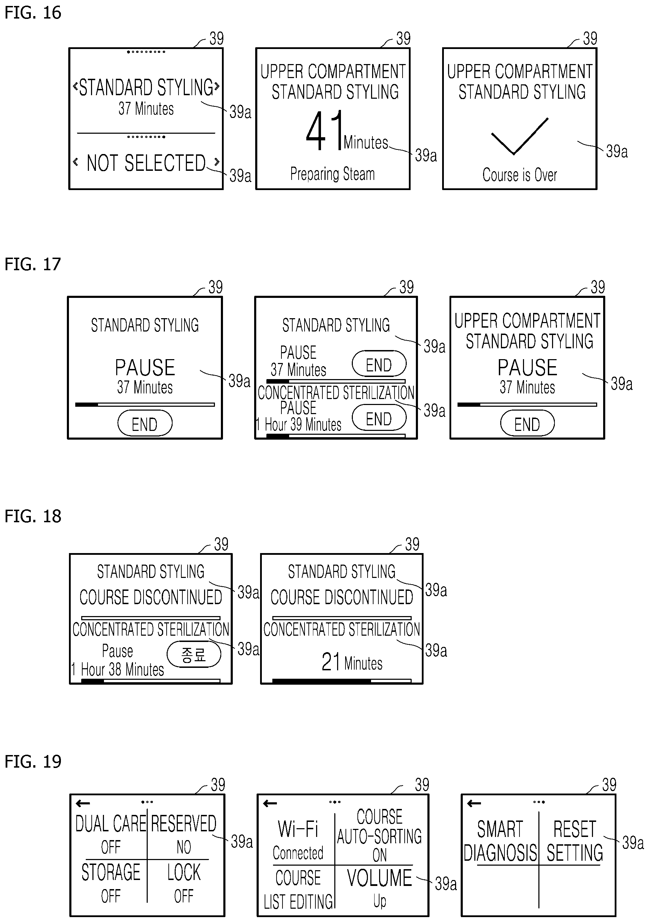

FIG. 14 is a view illustrating, by way of an example, the state in which general care and dual care are selected through the setting UI 38c in the shoe care device 1 according to one embodiment of the present invention. FIGS. 15 and 16 are views illustrating, by way of an example, UIs which are operated in a dual care mode in the shoe care device 1 according to one embodiment of the present invention. FIGS. 17 and 18 are views illustrating, by way of an example, the state in which the operation UI 38b is selected while a processing stroke is being executed in the shoe care device 1 according to one embodiment of the present invention.

In the shoe care device 1 according to one embodiment of the present invention, a plurality of inner cabinets 100 may be disposed, and a connection path F10 may be provided for each inner cabinet 100. In this case, the controller 10 may control the second control UI 39a for each inner cabinet 100 to be individually displayed on the main layer 39.

In this regard, the configuration in which the inner cabinets 100 are disposed up and down is illustrated, but is not necessarily limited thereto. If necessary, the inner cabinets 100 may be arranged front and rear, left and right, up and down, or in a combination form thereof.

In addition, the connection paths F10 may be individually installed in the inner cabinets 100, respectively, so that a processing stroke for shoes can be executed independently in each inner cabinet 100.

Meanwhile, in the shoe care device 1 in which a plurality of inner cabinets 100 are arranged as described above, all the inner cabinets 100 may be unified and operated in one processing stroke depending on the user's selection (which may be referred to as "general care").

Alternatively, in the shoe care device 1 in which a plurality of inner cabinets 100 are arranged, inner cabinets 100 may be operated independently in different processing strokes, respectively, depending on the user's selection (which may be referred to as "dual care").

When the shoe care device 1 is operated in a general care mode, the main layer 39 may be controlled to display second control UIs 39a for all the processing strokes (see FIG. 13).

On the other hand, when the shoe care device 1 is operated in a dual care mode, the second control UIs 39a for processing strokes of respective inner cabinets 100 may be controlled to be individually displayed on the main layer 39 (see FIG. 15).

Accordingly, it may be possible to check all processing stroke states for the plurality of inner cabinets 100 from one control panel 33 (especially, the main layer 39).

In this way, in the shoe care device 1 according to the present embodiment, since a plurality of inner cabinets 100 are arranged and UIs for each inner cabinet 100 are individually displayed on the main layer 39, the states of respective inner cabinets 100 which execute processing strokes independently can be easily checked.

In the shoe care device 1 according to one embodiment of the present invention, the plurality of inner cabinets 100 may be stacked in the vertical direction. In this case, the controller 10 may control the second control UI 39a for respective inner cabinets 100 to be divided and displayed up and down on the main layer 39.

That is, a second control UI 39a displayed in the upper portion of the main layer 39 may display the state of the processing stroke of the inner cabinet 100 disposed at the upper side, and a second control UI 39a displayed in the lower portion of the main layer 39 may display the state of the processing stroke of the inner cabinet 100 disposed at the lower side.

Therefore, the user may easily identify and operate the state of the processing stroke for each of the inner cabinet 100 by checking the contents divided and displayed up and down on the control panel 33 (especially, the main layer 39).

In this way, in the shoe care device 1 according to the present embodiment, since the plurality of inner cabinets 100 are arranged in the vertical direction, and the UIs for each inner cabinet 100 are divided and displayed up and down on the main layer 39, the state of each of the inner cabinets 100 arranged up and down can be intuitively identified.

In the shoe care device 1 according to one embodiment of the present invention, the first control UIs 38a, 38b, 38c, and 38d may include a setting UI 38c through which the settings of the shoe care device 1 can be selected. In this case, when the setting UI 38c is selected, the controller 10 may control settings for independently executing a processing stroke in each inner cabinet 100 to be displayed on the main layer 39.

That is, as illustrated in FIG. 14, when the user selects the setting UI 38c among the first control UIs 38a, 38b, 38c, and 38d, a window for selecting whether to perform dual care is displayed on the main layer 39.

In this case, the user may select whether to operate the shoe care device 1 in a general care mode or a dual care mode by operating a dual care selection window displayed on the main layer 39.

In this way, in the shoe care device 1 according to the present embodiment, since the setting UI 38c is selected to check on the main layer 39 whether the processing strokes of respective inner cabinets 100 will be executed independently of each other, the utilization of the shoe care device 1 can be increased by selectively using general care and dual care.

In the shoe care device 1 according to one embodiment of the present invention, the controller 10 may control at least one of stroke type, remaining time, progress rate, and detailed stroke information to be displayed on the main layer 39 depending on the second control UI 39a selected for each inner cabinet 100.

That is, as illustrated in FIGS. 13 and 15, depending on the type of the processing stroke selected by the user, the second control UI 39a may display the stroke type so that the user can check it. At this time, the detailed stroke information about the corresponding processing stroke may be additionally displayed together with the stroke type to assist the user in making a selection.

In this state, when a few seconds (e.g., 2 seconds) elapse, instead of the detailed stroke information, the time required for the corresponding processing stroke may be displayed together with the stroke type to assist the user in making a selection.

In addition, when the user selects the operation UI 38b and the corresponding processing stroke is initiated, the displayed time, which indicates the remaining time, may be counted, and the progress rate of the processing stroke may be displayed together in a bar-type graph or the like.

In this way, in the shoe care device 1 according to the present embodiment, since at least one of the stroke type, remaining time, progress rate, and detailed stroke information for each inner cabinet 100 is displayed on the main layer 39, the user may more easily check the state of each inner cabinet 100.

In the shoe care device 1 according to one embodiment of the present invention, the controller 10 may control the second control UI 39a for one inner cabinet 100, of which the processing stroke was completed first, to be displayed on the main layer 39 as the completed state.

As described above, in the case of dual care in which the processing strokes of respective inner cabinets 100 are performed independently of each other, the start time pints of the processing strokes may be set to be the same.

Accordingly, since the completion time points of different processing strokes are different, one inner cabinet 100 may complete the processing stroke before the remaining inner cabinet 100.

Accordingly, for one inner cabinet 100 for which the processing stroke was completed first, the second control UI 39a may be displayed as the completed state. In addition, this display of completed state may be maintained until the processing stroke of the remaining one inner cabinet 100 is completed.

In this way, it may be desirable to allow the user to check in real time that one inner cabinet 100 has completed the processing stroke and the remaining one inner cabinet 100 is still executing the processing stroke so that the user can refer to the situation when inputting a subsequent control signal to the shoe care device 1.

In this way, in the shoe care device 1 according to the present embodiment, since the state of the inner cabinet 100 for which the processing stroke was completed first is displayed on the main layer 39 until all the processing strokes are completed, it may be easy to check the state in which only one inner cabinet 100 has completed the processing stroke in dual care.

In the shoe care device 1 according to one embodiment of the present invention, when the second control UIs 39a selected for respective inner cabinets 100 are the same, the controller 10 may control the respective second control UIs 39a to be integrated and displayed on the main layer 39.

As described above, when the user selects dual care through the setting UI 38c, but the second control UIs 39a selected for respective inner cabinets 100 are the same, it may be actually necessary to proceed with general care.

That is, since the processing strokes for respective inner cabinets 100 are the same, it may be said that the start time points, stroke types, completion time points of the processing strokes are all the same, and there is no need to individually display the second control UIs 39a.

In this way, in the shoe care device 1 according to this embodiment, when the processing strokes of respective inner cabinets 100 are the same, the UIs for respective inner cabinets 100 are integrated and displayed on the main layer 39. Therefore, it may be easy to check the state in which the processing strokes are set to dual care but are actually in a general care state.

Meanwhile, as illustrated in FIG. 16, even when dual care is selected, a processing stroke may be executed only in a desired inner cabinet 100. That is, the second control UI 39a for one inner cabinet 100 may be selected as being "not selected" to prevent the processing process for the corresponding inner cabinet 100 from being operated.

Even in this case, since there is no need to display the second control UI 39a individually, all of the second control UI 39a for the inner cabinets 100 in operation may be displayed throughout the main layer 39. However, it may be desirable to display "top" or "bottom" together on the second control UIs 39a so that the user can determine which inner cabinet 100 is in operation.

In the shoe care device 1 according to one embodiment of the present invention, when no control signal is input to the control panel 33 during a processing stroke for a set period of time, the controller 10 may control the remaining time to be displayed in an enlarged format on the main layer 39.

As described above, when the user selects a processing stroke for an inner cabinet 100 and the corresponding stroke is being executed, input of additional control signals may not be made other than temporarily stopping or forcibly terminate the operation of the shoe care device 1.

That is, while a processing stroke is being executed normally, it is common that input of additional control signals is not made on the control panel 33 during a set period of time. In this case, it is common that a user usually performs other tasks relatively far away from the shoe care device 1 rather than being close to the shoe care device 1.

Therefore, it may be desirable to allow the user to check the operating state of the shoe care device 1 from a distance while the processing stroke is being executed normally.

To this end, the remaining time may be controlled to be displayed in an enlarged format on the main layer 39, so that the user can determine from a distance how much time is left until the corresponding stroke is completed.

In this case, in the dual care state, the remaining time of the inner cabinet 100, of which the processing stroke is completed relatively late, may be controlled to be displayed in an enlarged format on the main layer 39.

In this way, in the shoe care device 1 according to the present embodiment, since the remaining time is displayed in an enlarged format on the main layer when there is no additional operation during the set period of time while the processing stroke is being executed, the user may easily check the state of each inner cabinet 100 even from a distance.

Meanwhile, when the remaining time is displayed in an enlarged format on the main layer 39 and an additional control signal is input to the control panel 33, it means that the user intends to approach the shoe care device 1 and perform a separate operation. In this regard, the main layer 39 may be controlled to display the second control UIs 39a in a normal state.

In the shoe care device 1 according to one embodiment of the present invention, the controller 10 can control the types of second control UIs 39a to be changed and displayed on the main layer 39 by a sliding method.

Since the types of second control UIs 39a are changed according to the user's intention, it may be convenient to allow various types of UIs to be changed in a sliding manner on the main layer 39 of a certain area.

In this case, the sliding manner may be implemented by the user swiping the screen of the main layer 39 or tapping the left and right buttons displayed on the screen of the main layer 39, as described above.

In this way, in the shoe care device 1 according to the present embodiment, since the type of a UI for each inner cabinet 100 is checked on the main layer 39 in a sliding manner, the UI can be effectively operated on the main layer 39.

In the shoe care device 1 according to one embodiment of the present invention, the first control UIs 38a, 38b, 38c, and 38d may further include a viewing UI 38d through which the stroke display of the shoe care device 1 can be selected. In this case, when the viewing UI 38d is selected, the controller 10 may control a plurality of types of second control UIs 39a for respective inner cabinets 100 to be displayed on the main layer 39.

As described above, the user may select the viewing UI 38d to cause a plurality of second control UIs 39a for respective processing strokes to be displayed on the main layer 39 at once, and then select and change one of the second control UIs into a second control UI 39a for a specific processing stroke.

In this case, as illustrated in FIG. 15, in a dual care state, when the user selects the viewing UI 38d, control may be performed to preferentially select which compartment of the inner cabinet 100 the processing stroke is for.

In this way, in the shoe care device 1 according to the present embodiment, since the viewing UI 38d is selected to check a plurality of types of UIs for respective inner cabinets 100 on the main layer 39, the plurality of UIs may be checked on the main layer 39 at once.

In the shoe care device 1 according to one embodiment of the present invention, the first control UIs 38a, 38b, 38c, and 38d may further include an operation UI 38b through which whether to operate the shoe care device 1 is selectable. In this case, when the operation UI 38b is selected while a processing stroke is being executed, the controller 10 may control a termination selection window to be displayed on the main layer 39.

As described above, while a processing stroke is being executed normally, it is common that input of additional control signals is not made on the control panel 33 during a set period of time. However, when the user wants to temporarily stop or forcibly terminate the operation of the shoe care device 1, the user may select the operation UI 38b.

In this case, as illustrated in FIG. 17, the second control UI 39a of the corresponding inner cabinet 100 may display a termination selection window such that the user selects whether to temporarily stop or forcibly terminate the operation of the shoe care device 1.

In addition, as illustrated in FIG. 18, when the user selects one of termination selection windows, the processing stroke of the corresponding inner cabinet 100 may be stopped and forcibly terminated. In this state, when the user selects the operation UI 38b again, only the processing stroke for the remaining inner cabinet 100 for which the termination selection field has not been selected can be resumed.

In this way, in the shoe care device 1 according to the present embodiment, while the processing stroke is being executed, the operation UI 38b may be selected to check on the main layer 39 whether to immediately terminate a processing stroke. Thus, if necessary, the operation of the shoe care device 1 may be terminated immediately while the processing stroke is being executed.

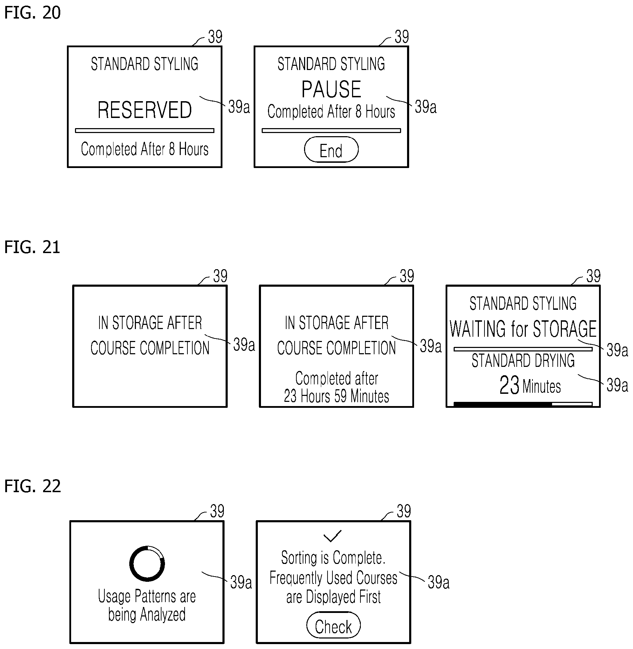

FIG. 19 is a view illustrating, by way of an example, the state of the main layer 39 displayed when the setting UI 38c is selected in the shoe care device 1 according to one embodiment of the present invention. FIG. 20 is a view illustrating, by way of an example, the state in which a completion time point of a processing stroke is reserved through the setting UI 38c in the shoe care device 1 according to one embodiment of the present invention. FIG. 21 is a view illustrating, by way of an example, the state in which shoes are stored after completion of a processing stroke through the setting UI 38c in the shoe care device 1 according to one embodiment of the present invention.

In the shoe care device 1 according to one embodiment of the present invention, the first control UIs 38a, 38b, 38c, and 38d may include a setting UI 38c through which the settings of the shoe care device 1 can be selected. In this case, when the setting UI 38c is selected, the controller 10 may control the setting for entering the completion time point of the processing intended by the user to be displayed on the main layer 39.

As illustrates in FIG. 19, when the user selects the setting UI 38c, various setting functions for the shoe care device 1 may be displayed. Among the setting functions, the user may select a reservation function to intend the processing stroke to be completed at the set completion time point.

That is, as illustrated in FIG. 20, the user may set the processing stroke to be completed after a specific period of time through the reservation function.

In this case, in the dual care state, the completion time point set by the user may be calculated and operated with reference to the inner cabinet 100, which completes the processing stroke relatively late.

In this way, in the shoe care device 1 according to the present embodiment, since the setting UI 38c may be selected to check the completion time point of the processing stroke to be reserved on the main layer 39, the shoe care device 1 may be operated according to the completion time point of the processing stroke intended by the user.

In the shoe care device 1 according to one embodiment of the present invention, the first control UIs 38a, 38b, 38c, and 38d may include a setting UI 38c through which the settings of the shoe care device 1 can be selected. In this case, when the setting UI 39a is selected, the controller 10 may control the second control UI 39a to be displayed as a storage state on the main layer 39 for a set period of time after the processing stroke is completed.

As illustrates in FIG. 19, when the user selects the setting UI 38c, various setting functions for the shoe care device 1 may be displayed. Among these functions, the user may select a storage function to intend that the storage state is maintained for a specific period of time (e.g., up to 24 hours) after the processing stroke is completed.

That is, as illustrated in FIG. 21, even when the processing stroke is completed through the storage function, the user may set the storage function such that shoes are stored in an optimal condition within the inner cabinet 100 for a specific period of time.

In this case, in the dual care state, the storage state may be maintained for a specific period of time from the time point at which the processing strokes of all of the inner cabinets 100 are completed, and the second control UI 39a for the inner cabinet 100 for which the processing stroke was completed first may be displayed as a storage standby state.

In addition, when the user operates the power UI 38a or opens an inner cabinet 100 while the storage function is in operation, it may be considered that there is no intention to maintain the storage function any longer, and the operation and power supply of the shoe care device 1 may be terminated.

In this way, in the shoe care device 1 according to the present embodiment, the setting UI 38c may be selected to check on the main layer 39 whether to maintain the setting UI as the storage state after a processing stroke is completed. Thus, even after the processing stroke is completed for shoes, the shoe care device 1 may be operated to store the shoes for a set period of time.

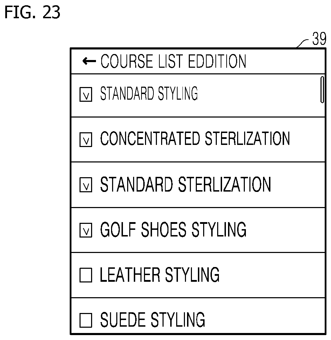

FIG. 22 is a view illustrating, by way of an example, the state in which the types of UIs are arranged depending on frequencies of processing strokes in the shoe care device 1 according to one embodiment of the present invention. FIG. 23 is a view illustrating, by way of an example, the state in which a UI selection list is displayed on the main layer 39 in the shoe care device 1 according to one embodiment of the present invention.

In the shoe care device 1 according to one embodiment of the present invention, the controller 10 may control the types of second control UIs 39a to be sequentially arranged by reflecting processing strokes executed a set number of times or more.

As described above, the user may select the second control UI 39a for an intended processing stroke through a sliding manner, viewing UI selection, or the like.

In this case, it may be more convenient for the user to preferentially display the second control user 39a for the user's most preferred processing stroke.

Therefore, as illustrated in FIG. 22, in order to ensure reliability, it may be desirable to arrange the second control UIs such that the second control UI 39a for the most frequent processing stroke is displayed preferentially by reflecting the processing strokes executed a set number (e.g., 20 times) or more.

In this way, in the shoe care device 1 according to the present embodiment, since the types of UIs are sequentially arranged depending on the frequencies of processing strokes executed a set number of times or more, the UIs for processing strokes frequently used by the user may be displayed preferentially.

In the shoe care device 1 according to one embodiment of the present invention, the first control UIs 38a, 38b, 38c, and 38d may include a setting UI 38c through which the settings of the shoe care device 1 can be selected. In this case, when the setting UI 38c is selected, the controller 10 may control the main layer 39 to display a list from which the type of the second control UI 39a can be selected.

As illustrates in FIG. 19, when the user selects the setting UI 38c, various setting functions for the shoe care device 1 may be displayed. Among the setting functions, the user may select a course list editing function to cause relatively preferred processing strokes to be exposed preferentially.

That is, as illustrated in FIG. 23, the user may cause processing strokes checked through the course list editing function to be displayed on the main layer 39 and cause unchecked processing strokes not to be displayed.

In this way, in the shoe care device 1 according to the present embodiment, since the setting UI 38c is selected to check the UI selection list on the main layer 39, only the UIs for processing strokes required by the user may be displayed on the main layer 39.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### - Description of Reference Numerals-

| | | | |
|---|---|---|---|
| 1: | shoe care device | 2a: | first management device |
| 2b: | second management device | 3: | cabinet |
| 10: | controller | 20: | outer cabinet |
| 30: | door | 33: | control panel |
| 40: | main shelf | 50: | machine room |
| 60: | water supply tank | 70: | drain tank |
| 100, 100a, 100b: | inner cabinet | 101: | accommodation space |
| 200: | module housing | 201a: | module opening |
| 201: | module case | 202: | module cover |
| 203: | outlet | 204: | steam inlet |
| 210: | module chamber | 211: | suction module chamber |
| 212: | first module chamber | 213: | second module chamber |
| 214: | third module chamber | 310: | blowing part |
| 320: | heating part | 330: | dehumidifying part |
| 350: | damper | 361: | first sensor |
| 362: | second sensor | 363: | third sensor |
| 370: | dry air duct | 400: | condenser |
| 600: | sump | 700: | steam part |
| 810: | nozzle duct | 820: | nozzles |
| DM, DM1, DM2: | drying module | F10: | connection path |
| F10a: | conversion flow path | F10b: | drying flow path |
| F20: | regeneration path | RP: | reference plane |
| X: | first direction | Y: | second direction |
| Z: | third direction | | |

### INDUSTRIAL APPLICABILITY

By at least one of exemplary embodiments of the present invention, a dehumidifying part is disposed in a module chamber not only to collect moisture and bacteria in a ventilation air but also to regenerate the dehumidifying part by heating the dehumidifying part in the module chamber, thereby maintaining proper shoes treatment performance all the times.

In addition, by at least one of exemplary embodiments of the present invention, a connection path through which air circulates is formed between a outlet and a nozzle respectively disposed inside an inner cabinet, thereby preventing the air used for dehumidifying and deodorizing shoes from being exposed to the user.

In addition, by at least one of exemplary embodiments of the present invention, when a user inputs a control signal for a processing stroke to the control panel, the display state of information output through the control panel is controlled accordingly. Thus, the user may easily check the state of the shoe care device and to smoothly input a control signal for an intended processing stroke.

In addition, by at least one of exemplary embodiments of the present invention, since a plurality of inner cabinets are arranged and UIs for respective inner cabinets are individually displayed on the main layer, it may be easy to check the state of each of the inner cabinets, which execute processing strokes independently of each other.

In addition, by at least one of exemplary embodiments of the present invention, since a plurality of inner cabinets are arranged up and down, and the UIs for respective inner cabinets are divided and displayed up and down on the main layer, the state of each of the inner cabinets arranged up and down may be intuitively determined.

In addition, by at least one of exemplary embodiments of the present invention, since a setting UI is selected to check on the main layer whether the processing strokes of respective inner cabinets will be performed independently of each other, the usability of the shoe care device may be increased by selectively using general care and dual care.

In addition, by at least one of exemplary embodiments of the present invention, since at least one of the stroke type, remaining time, progress rate, and detailed stroke information for each inner cabinet is displayed on the main layer, a user may more easily check the state of each inner cabinet.

In addition, by at least one of exemplary embodiments of the present invention, since the state of the inner cabinet for which the processing stroke was completed first is displayed on the main layer until all of the processing strokes are completed, it may be easy to check the state in which the processing stroke of only one inner cabinet is completed in dual care.

In addition, by at least one of exemplary embodiments of the present invention, when the processing strokes of respective inner cabinets are the same, the UIs for respective inner cabinets are integrated and displayed on the main layer. Therefore, it may be easy to check the state in which the processing strokes are set to dual care but are actually in the state of general care.

In addition, by at least one of exemplary embodiments of the present invention, when there is no additional operation during a set period of time while a processing stroke is being performed, the remaining time is displayed in an enlarged format on the main layer. Thus, a user may easily check the state of each inner cabinet even from a distance.

In addition, by at least one of exemplary embodiments of the present invention, since the type of a UI for each inner cabinet is checked on the main layer in a sliding manner, the UI may be effectively operated on the main layer.

In addition, by at least one of exemplary embodiments of the present invention, since a viewing UI may be selected to check a plurality of types of UIs for respective inner cabinets on the main layer, a plurality of UIs may be checked on the main layer at once.

In addition, by at least one of exemplary embodiments of the present invention, since an operation UI may be selected to check whether to immediately terminate the processing stroke while a processing stroke is being executed, the operation of the shoe care device may be terminated immediately while the processing stroke is being executed, if necessary.

In addition, by at least one of exemplary embodiments of the present invention, since a setting UI is selected to check the completion time point of a processing stroke to be reserved on the main layer, the shoe care device may be operated according to the completion time point of the processing stroke intended by the user.

In addition, by at least one of exemplary embodiments of the present invention, since the setting UI is selected to check on the main layer whether to maintain the setting UI as the storage state after a processing stroke is completed, the shoe care device may be operated to store the shoes for a set period of time even after the processing stroke is completed for shoes.

In addition, by at least one of exemplary embodiments of the present invention, since the types of UIs are sequentially arranged depending on the frequencies of processing strokes executed a set number of times or more, the UIs for processing strokes frequently used by the user may be displayed preferentially.

In addition, by at least one of exemplary embodiments of the present invention, since a UI selection list is checked on the main layer by selecting a setting UI, only the UIs for processing strokes required by the user may be displayed on the main layer.

## Claims

1. A shoe care device configured to execute a processing stroke on a shoe accommodated in an accommodation space of an inner cabinet, the shoe care device comprising:
a connection path configured to provide a flow path into which air from the accommodation space is introduced and then discharged back into the accommodation space;
a blowing part disposed in the connection path and configured to blow air;
a dehumidifying part disposed in the connection path and configured to dehumidify the air;
a steam part configured to supply steam to the inner cabinet;
a control panel disposed outside the shoe care device so that a control signal for the processing process can be input by a user; and
a controller configured to control a display state of information output through the control panel depending on a type of the processing stroke,
wherein the control panel comprises:
a sub-layer where a first control UI, of which display-on/off can be controlled, is disposed; and
a main layer where a second control UI, of which display-on/off and type can be controlled, is disposed.

2. The shoe care device of claim 1, wherein a plurality of inner cabinets are arranged, and the connection path is provided in each of the inner cabinets, and
wherein the controller controls the second control UI for each of the inner cabinets to be individually displayed on the main layer.

3. The shoe care device of claim 2, wherein the plurality of inner cabinets are stacked in a vertical direction, and
wherein the controller controls the second control UI for each of the inner cabinets to be divided and displayed up and down on the main layer.

4. The shoe care device of claim 2, wherein the first control UI comprises a setting UI through which a setting of the shoe care device is selectable, and
wherein the controller controls a setting for independently executing the processing stroke in each inner cabinet to be displayed on the main layer when the setting UI is selected.

5. The shoe care device of claim 4, wherein the controller controls at least one of a stroke type, a remaining time, a progress rate, and detailed stroke information to be displayed on the main layer according to the second control UI selected for each of the inner cabinets.

6. The shoe care device of claim 5, wherein the controller controls the second control UI for one of the inner cabinets for which the processing stroke was completed first to be displayed as a completion state on the main layer.

7. The shoe care device of claim 5, wherein, when the second control UIs selected for respective inner cabinets are equal to each other, the controller controls each of the second control UIs to be integrated and displayed on the main layer.

8. The shoe care device of claim 5, wherein, when no control signal is input to the control panel for a set period of time while the processing stroke is being executed, the controller controls a remaining time to be displayed in an enlarged format on the main layer.

9. The shoe care device of claim 5, wherein the controller controls a type of the second control UI to be changed and displayed on the main layer in a sliding manner.

10. The shoe care device of claim 5, wherein the first control UI further comprises a viewing UI through which a stroke display of the shoe care device is selectable, and
wherein, when the viewing UI is selected, the controller controls a plurality of types of second control UIs for respective inner cabinets to be displayed on the main layer.

11. The shoe care device of claim 5, wherein the first control UI further comprises an operation UI through which whether to operate the shoe care device is selectable, and
wherein, when the operation UI is selected during the processing process, the controller controls a termination selection window to be displayed on the main layer.

12. The shoe care device of claim 2, wherein the first control UI comprises a setting UI through which a setting of the shoe care device is selectable, and
wherein, when the setting UI is selected, the controller controls a setting for inputting a completion time point of the processing stroke intended by a user to be displayed on the main layer.

13. The shoe care device of claim 2, wherein the first control UI comprises a setting UI through which a setting of the shoe care device is selectable, and
wherein, when the setting UI is selected, the controller controls the second control UI to be displayed as a storage state on the main layer for a set period of time after completion of the processing stroke.

14. The shoe care device of claim 2, wherein the controller controls types of second control UIs to be sequentially arranged by reflecting the processing strokes executed the set number of times or more.

15. The shoe care device of claim 2, wherein the first control UI comprises a setting UI through which a setting of the shoe care device is selectable, and
wherein, when the setting UI is selected, the controller controls a list from which the type of the second control UI is selectable to be displayed on the main layer.
